# EUROPÄISCHE PATENTSCHRIFT

(11) **EP 2 879 722 B1**
(45) Veröffentlichungstag und Bekanntmachung des Hinweises auf die Patenterteilung: **23.03.2016**
(21) Anmeldenummer: 13802548.1
(22) Anmeldetag: 15.11.2013
(51) Int. Cl.: A61L 2/20

(54) **ISOLATOR MIT KATALYTISCHEM DESINFEKTIONSMITTELABBAU**
ISOLATOR HAVING CATALYTIC DISINFECTANT DECOMPOSITION
ISOLATEUR À DÉGRADATION CATALYTIQUE D'AGENTS DÉSINFECTANTS

(30) Priorität: 20.11.2012 DE 102012111148
(43) Veröffentlichungstag der Anmeldung: 10.06.2015
(60) Teilanmeldung: 15196456.6
(73) Patentinhaber: Metall + Plastic GmbH, 78315 Radolfzell (DE)
(72) Erfinder: VON STENGLIN, Christoph, 78315 Radolfzell (DE)
(74) Vertreter: Wagner, Kilian
(86) Internationale Anmeldenummer: PCT/EP2013/073934
(87) Internationale Veröffentlichungsnummer: WO 2014/079779

(56) Entgegenhaltungen:
- EP-A1- 2 210 618
- EP-A1- 2 335 741
- WO-A1-2004/004790
- WO-A1-2011/085735
- CH-A5- 689 178
- DE-A1- 4 340 788

## Beschreibung

Die Erfindung betrifft eine Dekontaminationsanordnung, insbesondere für pharmatechnische Anwendungen, gemäß dem Oberbegriff des Anspruchs 1.

Eine gattungsbildende Dekontaminationsanordnung zeichnet sich dadurch aus, dass eine, bevorzugt einen Verdampfer, insbesondere für Wasserstoffperoxid umfassende Dekontaminationseinrichtung innerhalb eines oberhalb eines zu dekontaminierenden Raums angeordneten Umlufterzeugerraum angeordnet ist und/oder einen in den Umlufterzeugerraum ausmündenden Auslass für Dekontaminationsmittel, insbesondere gasförmiges Wasserperoxid aufweist.

In der Regel weist eine gattungsbildende Dekontaminationsanordnung einen Frischluftanschluss auf, über den konditionierte (getrocknete und temperierte) Frischluft zugeführt werden kann, um das Dekontaminationsmittel aus dem zu dekontaminierenden Raum nach erfolgter Dekontamination auszublasen, d.h. den Raum spülen zu können. Dieses sogenannte Freispolen nimmt einen erheblichen Zeitraum in Anspruch, der in Abhängigkeit der Größe des Isolators und der gewünschten, zu erzielenden (minimalen) Dekontaminationsmittelkonzentration von beispielsweise 1ppm oder weniger 3 bis 10 Stunden oder mehr betragen kann. Die in der Praxis zum Einsatz kommenden Frischluftkonditionierungseinrichtungen umfassen dabei meist ein Zuluftgebläse (Fristluftgebläse), welches üblicherweise derart ausgelegt Ist, dass mit diesem in dem zu dekontaminierendem Raum und allen damit strömungstechnisch verbundenen Räumen ein etwas 150-facher Luftwechsel pro Stunde realisierbar ist.

Es ist also etwa ein 150-faches Raumvolumen pro Stunde mittels des Frischluftgebläses zuführbar. Ferner umfasst eine gattungsbildende Dekontaminationsanordnung in dem Umtufterzeugerraum ein Gebläse, auch als Umluftgebläse bezeichnet, um zwischen dem Umluftraum und dem bevorzugt in der Projektionsebene darunter befindlichen zu dekontaminierenden Raum, insbesondere einem Manipulatorraum Gas, insbesondere Luft zu zirkulieren. Bei einer gattungsgemäßen Dekontaminationseinanordnung wird das Umluftgebläse zwingend benötigt, um eine ausreichende Menge an Dekontaminationsmittel aus dem Umlufterzeugerraum in den zu dekontaminierenden Raum zu überführen.

Eine gattungsgemäße Dekontaminationsanordnung unterscheidet sich von einer beispielsweise in der EP 0 604 925 A1 beschriebenen Dekontaminationsanordnung im Wesentlichen dadurch, dass bei der in der EP 0 604 925 A1 gezeigten Dekontaminationsanordnung die Dekontaminationseinrichtung (Wasserstoffperoxidverdampfer) außerhalb des Umlufterzeugerraums angeordnet ist und das Dekontaminationsmittel nicht unmittelbar an den Umlufterzeugerraum eingeleitet wird.

In der WO 2011/085735 A1 ist eine nicht gattungsgemäße Dekontaminationsanordnung beschrieben, die sich Im Wesentlichen dadurch von einer gattungsgemäßen Dekontaminationsanordnung unterscheidet, als dass der Wasserstoffperoxidverdampfer außerhalb des Umlufterzeugerraumes angeordnet ist und das Dekontaminationsmittel nicht in dem Umlufterzeugerraum sondern alternativ in den zu dekontaminierenden Raum oder einen zwischen Umlufterzeugerraum und zu dekontaminlerenden Raum angeordneten Zwischenraum eingeleitet wird. Bei einer derartigen Anordnung wird das Umluftgebläse nicht benötigt, um das dampfförmige Dekontaminationsmittel in ausreichender Menge in den zu dekontaminierenden Raum einzubringen.

Bei der aus der WO 2011/085735 A1 bekannten Vorrichtung wird die Freispülzeit in vorteilhafter Weise dadurch verkürzt, dass beim Freispülen das Dekontaminationsmittel mittels des Umluftgebläses durch einen im Umlufterzeugerraum angeordneten Katalysator gefördert wird. Einen Abbau von Dekontaminationsmittel während einer Konditionierungsphase, In der Dekontaminationsmittel zugeführt wird und/oder während einer Einwirkphase während derer das Dekontaminationsmittel seine dekontaminierende Wirkung entfaltet wird dadurch verhindert, dass das Umluftgebläse zumindest über den größten Zeitabschnitt der Konditionierungsphase bzw. der Einwirkphase ausgeschaltet wird oder bleibt.

Aus der US 6,010,400 A ist ebenfalls eine gattungsfremde Dekontaminationsanordnung ohne Umlufterzeugerraum bekannt. Das Umluftgebläse sitzt in einem Rohrsystem benachbart zu dem zu dekontaminierenden Raum. Mittels des Umluftgebläses kann das Dekontaminationsmittel durch einen mit Katalysatormaterial getränkten Filter hindurchgefördert werden, oder durch einen anderen Kanal an diesem vorbei. Das Gebläse sitzt in Förderrichtung vor dem Filter, so dass der Gasvolumenstrom durch den Filter mit Katalysatormaterial gedrückt wird.

Aus der JP 2011-167405 A ist es bekannt dekontaminationsmittelhaltiges Gas in einem ersten Betriebszustand an einem Katalysator vorbei und In einem zweiten Betriebszustand durch diesen hindurch zu fördern, wobei hierzu Gasleitmittel mittels eines aktiven Antriebs aktiv verstellt werden müssen.

Ausgehend von dem vorgenannten Stand der Technik liegt der Erfindung die Aufgabe zugrunde, eine gattungsgemäße Dekontaminationsanordnung mit einer vorzugsweise einen Verdampfer für die Dekontaminationsmittel umfassenden, in dem Umlufterzeugerraum angeordneten Dekontaminationseinrichtung und/oder einer einen in den Umluftezeugerraum ausmündenden Auslass für Dekontaminationsmittel aufweisenden Dekontaminationseinrichtung so weiter zu bilden, dass die Freispülzeit reduziert wird. Ferner besteht die Aufgabe darin, ein Verfahren zum Betreiben einer gattungsgemäßen Dekontaminationsanordnung mit dem Ziel der Reduzierung der Freispülzeit anzugeben.

Diese Aufgabe wird hinsichtlich der Dekontaminationsanordnung mit den Merkmalen des Anspruchs 1 und hinsichtlich des Verfahrens mit den Merkmalen des Anspruchs 12 gelöst. Vorteilhafte Weiterbildungen der Erfindung sind In den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

Zur Vermeidung von Wiederholungen sollen vorrichtungsgemäß offenbarte Merkmale als verfahrensgemäß offenbart gelten und beanspruchbar sein. Ebenso sollen verfahrensgemäß offenbarte Merkmale als vorrichtungsgemäß offenbart gelten und beanspruchbar sein.

Die Erfindung hat erkannt, dass es zur Reduzierung der Freispülzeit, wie dies beispielsweise in der gattungsfremden WO 2011/085735 A1 beschrieben ist, das Dekontaminationsmittel im Kreislauf durch einen Katalysator zur Aufspaltung des Dekontaminationsmittels zu fördern. Da bei einer gattungsgemäßen Dekontaminationsanordnung das Umluftgebläse zur Verteilung des dammförmigen Dekontaminationsmittels in Betrieb sein muss, aufgrund der Tatsache, dass sich die Dekontaminationseinrichtung, insbesondere ein Verdampfer unmittelbar im Umlufterzeugerraum befindet und/oder ein Auslass der Dekontaminationseinrichtung unmittelbar in den Umlufterzeugerraum ausmündet, ist eine Anordnung, wie in der WO 2011/085735 A1 beschrieben nicht ohne weiteres möglich, da vermieden werden muss, dass zumindest ein Großteil des Dekontaminationsmittels In der Einbringphase und/oder der Einwirkphase des Dekontaminationsmittels durch den Katalysator gefördert wird. Um dies zu realisieren und gleichzeitig einen Betrieb des mindestens einen Umluftgebläses während der Beschickung mit Dekontaminationsmittel, Insbesondere Dekontaminationsmitteldampf und/oder während der Einwirkphase aus Gründen einer gleichmäßigen Verteilung des Dekontaminationsmittels zu ermöglichen, schlägt die Erfindung weiter vor, dem Gebläse bevorzugt im Umlufterzeugerraum angeordnete Gasleitmittel zuzuordnen, die zwischen einem ersten und einem zweiten Betriebszustand umschaltbar sind, wobei die Gasleitmittel in dem ersten Betriebszustand, währenddessen bevorzugt Dekontaminationsmittel eingeleitet wird und/oder das Dekontaminationsmittel seine dekontaminierende Wirkung entfaltet zur Verteilung des von der Dekontaminationseinrichtung abgegebenen Dekontaminstionsmittels zumindest der größte Teil des Mittels des Gebläses (Umluftgebläse) geförderte Gasvolumenstrom am Katalysator vorbeigeleitet wird und wobei In dem zweiten Betriebszustand, währenddessen die Freispülung erfolgt, in dem das Dekontaminationsmittel im Katalysator aufgespalten wird, zumindest der größte Teil des Mittels des Gebläses geförderten Gasumluftvolumenstroms durch den Katalysator hindurch gefördert wird. Wie später noch erläutert werden wird gibt es im Hinblick auf die konkrete Ausbildung der Gasleitmittel unterschiedliche Möglichkeiten. Entscheidend ist Jedoch, dass sowohl Umluftgebläse als auch Dekontaminationsmitteleinrichtung und/oder Dekontaminationsmittel-Einrichtungsauslass gemeinsam in dem oberhalb des zu dekontaminierenden Raums angeordneten Umlufterzeugerraum angeordnet sind.

Bevorzugt kommt dabei ein Katalysator zum Einsatz, der nicht wie in der US 6,010,400 beschrieben aus einem mit Katalysator beschichteten bzw. getränkten Filter besteht, was mit Nachteilen theoretisch auch möglich wäre, sondern bevorzugt ein Katalysator, bei dem das Katalysatormaterial, insbesondere MnO₂, als Schüttgut in einem Behältnis vorliegt. Noch weiter bevorzugt kommt als Katalysator eine mit Katalysator beschichtete Trägerstruktur, insbesondere ein offenporiger Metallschaum, noch weiter bevorzugt ein Aluminium-, Nickel- oder Chromnickelschaum zum Einsatz, wie dieser in der WO 2011/085735 A1 beschrieben ist.

Besonders zweckmäßig ist es, wenn im Gasumluftvolumenstrom, insbesondere in Strömungsrichtung hinter dem Katalysator ein Hochleistungsschwebstofffilter, insbesondere ein Hepa-Filter und/oder ULPA-Filter angeordnet ist.

Die erfindungsgemäße Dekontaminationsanordnung zeichnet sich durch die Anordnung des Umluftgebläses im Umlufterzeugerraum bevorzugt dadurch aus, dass das Gas zur Erzeugung eines Umluftstroms aus dem Umlufterzeugerraum ansaugt wird. Mit anderen Worten ist eine Ansaugöffnung des Umluftgebläses nicht wie in der aus der vorgenannten US-Schrift US 6,010,400 bekannten und keinen Umlufterzeugerraum aufweisenden Dekontaminationsanordnung eine Ansaugöffnung für das Gebläse im Umlufterzeugerraum vorgesehen. Bevorzugt befindet sich zwischen Umlufterzeugerraum und zu dekontaminierendem Raum ein Zwischenraum, der von dem zu dekontaminierendem Raum über eine Membran zur Vergleichsmäßigung des Luftstroms getrennt ist.

Wesentlich ist, dass dem mindestens einen Gebläse (Umluftgebläse) eine Steuereinrichtung zugeordnet ist, wie das mindestens eine Gebläse derart ansteuernd ausgebildet ist, dass im ersten Betriebszustand ein kleinerer Gasvolumenstrom von dem mindestens einen Gebläse gefördert wird als Im zweiten Betriebszustand. Dies kann auf unterschiedliche Weise realisiert werden. So kann das mindestens eine Gebläse im ersten Betriebszustand, bei welchem kein oder zumindest weniger Dekontaminationsmittel durch den Katalysator gefördert werden soll als im zweiten Betriebszustand, mit einer geringeren Drehzahl betrieben werden. Zusätzlich oder alternativ kann der kleinere Gasvolumenstrom dadurch realisiert werden, dass im ersten Betriebszustand weniger (Umluft-) Gebläse betrieben werden als im zweiten Betriebszustand.

Ganz besonders bevorzugt ist es, wenn zur Umstellung zwischen den Betriebszuständen der Gasleitmittel kein zusätzlicher bzw. separater Aktuator vorgesehen ist, sondern wenn die Verstellung zwischen den Betriebszuständen durch Förderung eines unterschiedlichen Gasvolumenstroms mittels des mindestens einen Gebläses erfolgt. Dies kann bei der saugenden Variante dadurch realisiert werden, dass dem mindestens einen Gebläse ein Gasleitmittelabschnitt der Gasleitmittel, insbesondere ein Ansaugkanalabschnitt der Gasleitmittel zugeordnet ist, der von dem mindestens einen Gebläse im zweiten Betriebszustand, insbesondere entgegen der Kraft von Ruckstellmitteln, beispielsweise mindestens einer Feder, angesaugt wird bzw. ansaugbar ist, so dass das mindestens eine Gebläse, insbesondere über den Gasleitmittelabschnitt, gasleitend mit dem Katalysator verbunden ist bzw. wird, so dass im zweiten Betriebszustand mehr Gas, insbesondere mehr Dekontaminationsmittel durch den Katalysator gefördert wird als im ersten Betrlebszustand. Hierzu kann ein bevorzugt zum Einsatz kommender Ansaugkanalabschnitt, der mittels des Gebläses im zweiten Betriebszustand angesaugt wird, beispielsweise in der Art eines Faltenbalgs ausgebildet sein. Bevorzugt umfasst der Gasleitmittetabschnitt, insbesondere der Ansaugkanalabschnitt eine Ansaugfläche, die sich winklig, insbesondere senkrecht zur Strömungsrichtung des angesaugten Gasvolumenstroms erstreckt, um ein Ansaugen durch das mindestens eine Gebläse zu erleichtern.

Für den Fall einer drückenden Anordnung des Gebläses kann eine aktuatorfreie Umstellung der Gasleitmittel zwischen den Betriebszuständen dadurch erreicht werden, dass dem mindestens einen Gebläse ein Gasleitmittelabschnitt, insbesondere ein Ausblaskanalabschnitt der Gasleitmittel zugeordnet ist, der von dem mindestens einen Gebläse im zweiten Betriebszustand, insbesondere entgegen der Kraft von Rückstellmitteln, bevorzugt mindestens einer Feder wegdrückbar ist, insbesondere in Richtung Katalysator, so dass das mindestens eine Gebläse gasleitend mit dem Katalysator verbunden ist bzw. wird, um zumindest einen Großteil des geförderten Gasvolumens, insbesondere ein Dekontaminationsmittelgasstrom (Hauptvolumenstrom) durch den Katalysator hindurchzufördern bzw. zu drücken.

Bevorzugt weist der Gasleitmittelabschnitt, insbesondere der Ausblaskanalabschnitt hierzu eine Druckfläche auf, die sich winklig, insbesondere senkrecht zur Ausblasrichtung bzw. zum geförderten Gasvolumenstrom erstreckt, um von dem Gebläse bzw. dem Gasumluftvolumenstrom angeströmt zu werden.

Ganz besonders zweckmäßig ist es im Fall der Verwendung eines Ausblaskanalabschnittes diesen in der Art eines Faltenbalgs auszubilden.

In Weiterbildung der Erfindung ist mit Vorteil vorgesehen, dass das mindestens eine Gebläse (Umluftgebläse) je nach Ausgestaltung und Anordnung der Luftleitmittel in dem ersten Betriebszustand das Gas aus dem Umlufterzeugerraum am Katalysator vorbeisaugt oder, wenn sich der Katalysator in Strömungsrichtung befindet an diesem vorbeidrückt. Ebenso verhält es sich je nach Ausgestaltung und Anordnung der Gasleitmittel im zweiten Betriebszustand. Alternativ wird das Gas durch den Katalysator hindurchgesaugt oder durch diesen hindurchgedrückt.

Als besonders zweckmäßig hat es sich herausgestellt, die Gasleitmittel, insbesondere vollständig, innerhalb des oberhalb des zu dekontaminierenden Raums angeordneten Umlufterzeugerraums anzuordnen.

Wie eingangs erwähnt zeichnet sich die erfindungsgemäße Dekontaminationsanordnung dadurch aus, dass zum einen die Dekontaminationseinrichtung und/oder ihr Auslass im Umlufterzeugerraum angeordnet sind/ist bzw. ausmündet und dass das Gebläse (Umluftgebläse) Gas unmittelbar aus dem Umlufterzeugerraum ansaugt. Bevorzugt ist es nun, dass die Auslassöffnung der Dekontaminationsmittelelnrichtung nicht über ein Rohr mit einer Ansaugöffnung des Gebläses verbunden ist, sondern dass beide frei beabstandet innerhalb des Umlufterzeugerraums angeordnet sind, also weiter außen nur von einer Hülle des Umlufterzeugerraums umgeben sind.

Je nach Ausbildung und Anordnung des Gebläses relativ zu den Gasleitmitteln, arbeitet das Gebläse in Bezug auf den Katalysator saugend oder blasend bzw. drückend. Für die eine Ausführungsform ist es bevorzugt, wenn eine erste Ansaugöffnung der Gasleitmittel, durch die Gas mittels des mindestens einen Gebläses im ersten Betriebszustand (aus dem Umlufterzeugerraum) ansaugbar ist und/oder eine zweite Ansaugöffnung, durch die Gas mittels des mindestens einen Gebläses im zweiten Betriebszustand (durch den Katalysator hindurch) ansaugbar ist, im Umlufterzeugerraum ausgebildet sind/ist.

Bei der anderen Alternative, bei der das Gebläse im zweiten Betriebszustand Gas durch den Katalysator bläst ist es bevorzugt, wenn eine erste Auslassöffnung der Gasleitmittel, durch die Gas mittels des mindestens einen Gebläses im ersten Betriebszustand ausblasbar ist und/oder eine zweite Auslassöffnung, durch die Gas mittels des mindestens einen Gebläses im zweiten Betriebszustand (in Richtung Katalysator) ausblasbar ist, im Umlufterzeugerraum ausgebildet sind/ist.

Als besonders zweckmäßig hat es sich herausgestellt, wenn bei der Dekontaminationsanordnung der zu dekontaminierende Raum nicht oder nicht nur über mindestens eine Rohrleitung sondern über mindestens einen zwischen zwei Doppeiglasscheiben gebildeten Kanal verbunden ist, durch den hindurch von außen bevorzugt das innere des zu dekontaminlerenden Raums sichtbar ist.

Die Erfindung führt auch auf ein Verfahren zum Betreiben einer erfindungsgemäßen Dekontaminationsanordnung, wobei sich das Verfahren dadurch auszeichnet, dass in einem ersten Zustand ein von dem mindestens einen Gebläse (Umluftgebläse) geförderter Gasvolumenstrom, zumindest größtenteils (Hauptvolumenstrom), an dem im Umlufterzeugerraum angeordneten Katalysator vorbeigefördert wird und, wobei im ersten Betriebszustand bevorzugt Dekontamlnationsmittel in den Umlufterzeugerraum eingeführt wird und/oder das Dekontaminationsmittel einwirkt und dass in einem zweiten Betriebszustand zur Aufspaltung des Dekontaminationsmittels, also zum Freispülen der Vorrichtung zumindest der größte Teil (Hauptvolumenstrom) des Mittels des Gebläses geförderten Gasvolumenstroms durch den mindestens einen Katalysator hindurchgefördert wird.

Besonders zweckmäßig ist es dabei, wenn von dem mindestens einen Gebläse im zweiten Betriebszustand ein größerer Gasvolumenstrom als im ersten Betriebszustand gefördert wird. Noch welter bevorzugt ist es, wenn die Umstellung zwischen den Betriebszuständen durch das Gebläse erfolgt, indem durch den größeren Gasvolumenstrom im zweiten Betriebszustand ein Gasleitmittelabschnitt, insbesondere ein Kanalabschnitt in Richtung Gebläse angesaugt, oder alternativ von dem Gebläse weggedrückt wird und somit Jedenfalls sichergestellt wird, dass der gesaugte oder ausgeblasene Gasvolumenstrom zumindest größtenteils durch den mindestens einen Katalysator strömt

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1:: in einer schematischen Darstellung den grundsätzlichen Aufbau eines möglichen Ausführungsbeispiels einer erfindungsgemäßen Dekontaminationsanordnung,
- Flg. 2a und Fig. 2b:: unterschiedliche Betriebszustände einer in Fig. 1 als Blackbox dargestellten Anordnung von Katalysator, Umluftgebläse und Gasleitmitteln, wobei das Gebläse im zweiten Betriebszustand (Fig. 2) den Gasumluftvolumenstrom durch den Katalysator saugt, und
- Fig. 3a und Fig. 3b:: eine alternative Anordnung der in Fig. 1 als Blackbox dargestellten Konfiguration von Katalysator, Gasleitmitteln und Gebläse, wobei hier das Gebläse in dem in Fig. 3b dargestellten zweiten Betriebszustand das aus dem Umlufterzeugerraum angesaugte Gas durch den Katalysator drückt.

In den Figuren sind gleiche Elemente und Elemente mit der gleichen Funktion mit den gleichen Bezugszeichen gekennzeichnet.

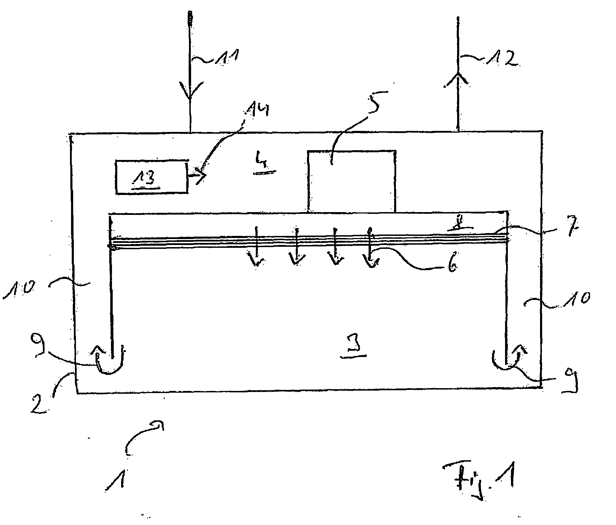

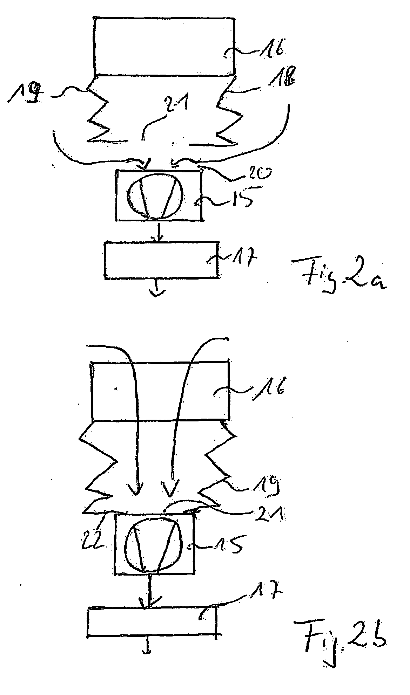

In Fig. 1 ist eine Dekontaminationsanordnung 1, umfassend einen Isolator 2 gezeigt. In diesem ist ein zu dekontaminierender Raum, beispielsweise ein Manipulatorraum vorgesehen. Oberhalb des zu dekontaminierenden Raums befindet sich ein Umlufterzeugerraum 4 mit einem als Umluftgebläse dienenden Gebläse, welches Teil einer "Blackbox" 5 ist, deren mögliche Ausgestaltungen anhand der Fig. 2a bis 3b im Folgenden noch erläutert werden. Teil der Blackbox 5 sind auch ein Katalysator zum Abbau von Dekontaminationsmittel sowie Gasleitmittel, die zwischen zwei Betriebszuständen umschaltbar sind. Fakultativ, jedoch bevorzugt sind auch Teil der Blackbox 5, wie später noch erläutert werden wird, Filtermittel, insbesondere in Form mindestens eines Hochleistungsschwebstofffilters, beispielsweise eines HEPA- und/oder ULPA Filters.

Das Gebläse ist so ausgelegt, dass mit diesem eine gleichmäßige Luftströmung 6 von oben nach unten in den zu dekontaminierenden Raum während des bestimmungsgemäßen Betriebs mit einer Strömungsgeschwindigkeit von etwa 0,45m/s erzeugbar ist. Das Gebläse erzeugt dabei zunächst eine turbulente Strömung, die mit einer Membran 7, ggf. in Kombination mit einem Hochleistungsschwebstofffilter in eine laminare Strömung umgewandelt wird. Der von dem Gebläse erzeugte Luftstrom gelangt in einen Zwischenraum 8, der nach unten von der vorerwähnten Membran 7 begrenzt ist. Die Membran 7 ist beispielsweise von einem Slebdruckgewebe gebildet. Die aus der Membran 7 nach unten strömende laminare Luftströmung gelangt in den zu dekontaminierenden Raum 3, strömt dort nach unten und dann in Pfeilrichtung 9 durch zwischen Doppelglasscheiben gebildete Strömungskanäle 10 innerhalb des Isolators 2 nach oben zurück in den Umlufterzeugerraum, aus dem sie von dem Gebläse wieder angesaugt wird.

In dem isolator 2, in dem konkreten Ausführungsbeispiel in dem oberen Umlufterzeugerraum 4 mündet eine Frischluftleitung 11 für konditionierte Luft, insbesondere während des Freispülvorgangs. Aus dem isolator 2, in dem konkreten Ausführungsbeispiel aus dem Umlufterzeugerraum 4 mündet eine Abluftleitung 12 hinaus. Der Frisrhluftleitung 11 ist eine nicht dar - gestellte Temperier- und Entfeuchtungseinrichtung vor- bzw. zugeordnet.

Innerhalb des Umlufterzeugerraums 4 befindet sich eine Dekontaminationseinrichtung 13 mit Verdampfer zum Verdampfen von Dekontaminationsmittel, insbesondere von Wasserstoffperoxid. Die Dekontaminationseinrichtung 13 weist einen Auslass 14 auf, der in den Umlufterzeugerraum 4 ausmündet und welcher innerhalb des Umlufterzeugerraums 4 beabstandet ist von einer in Fig. 1 nicht gezeigten Ansaugöffnung für das Gebläse.

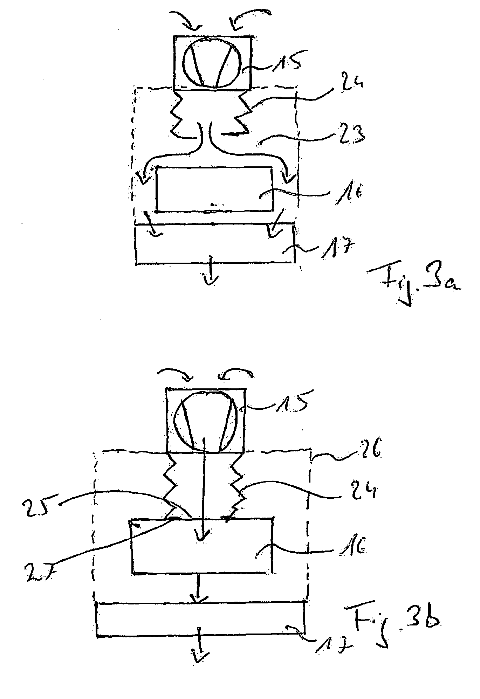

Im Folgenden werden anhand der Fig. 2a und 2b bzw. 3a und 3b Möglichkeiten zur Ausgestaltung der Anordnung von Umluftgebläse, Katalysator und Luftleitmitteln erläutert.

In Fig. 2a ist vorgenannte Anordnung in einem ersten Betriebszustand gezeigt, bei welchem durch mittels des Umluftgebläses 15 ein Hauptumluftvolumenstrom an einem Katalysator 16 zum Abbau von Dekontaminationsmittel vorbeigefördert wird, und zwar in Richtung zu dem zu dekontaminierenden Raum. Dem Umluftgebläse 15 ist in Förderrichtung ein Hochleistungsschwebstofffilter 17 nachgeordnet. Zusätzlich oder alternativ kann ein Hochleistungsschwebstofffilter auch in Strömungsrichtung vor dem Gebläse vorgesehen sein.

Damit der unmittelbar aus dem Umlufterzeugerraum 4 angesaugte Gasvolumenstrom größtenteils nicht durch den Katalysator 16 strömt sind Gasleitmtftel 18 vorgesehen, die in dem gezeigten Ausführungsbeispiel einen Ansaugkanal 19 in der Form eines Faltenbalgs umfassen. Das Umluftgebläse 15 wird bei einer vergleichsweise geringen Drehzahl betrieben, so dass der Ansaugkanal 18 nicht oder nicht ausreichend weit angesaugt wird. Hierdurch wird das Gas durch eine erste Ansaugöffnung 20 gesaugt, die sich zwischen dem Umluftgebläse 15 und dem Ansaugkanal 19 befindet. Durch eine zweite, von dem Ansaugkanal 19 begrenzte Ansaugöffnung 21 wird vergleichsweise wenig Gas angesaugt.

Im zweiten, in Fig. 2b dargestellten Betriebszustand wird. nun das Umluftgebläse 15 mit einer erhöhten Drehzahl betrieben (zusätzlich oder alterntiv können mehrere Umluftgebläse an- bzw. zugeschaltet werden), so dass der Ansaugkanal 19 in Richtung Umluftgebläse 15 angesaugt wird. Zu diesem Zweck weist der Ansaugkanal 19 Ansaugflächen 22 auf, die sich in dem gezeigten Ausführungsbeispiel winklig zur Ansaugrichtung des Umluftgebläses 15 erstrecken. Bei Förderung eines ausreichenden Gasvolumenstroms liegt der Ansaugkanal 19 am Gebläse 15 oder einem diesem zugeordneten Bauteil an, so dass der Ansaugkanal 19 das Gebläse 15 gasleitend mit dem Katalysator 16 verbindet. In dem gezeigten Ausführungsbeispiel ist in dem zweiten Betriebszustand die erste Ansaugöffnung 20 von dem Ansaugkanal 19 verschlossen und angesaugtes Gas strömt über den Katalysator 16 und die zweite Ansaugöffnung 21 zum Gebläse 15 und von diesem weiter in Richtung Filter und über diesen zur Membran 7 und in den zu dekontaminierenden Raum.

An dieser Stelle sei ausdrücklich erwähnt, dass es sich bei den Fig. 2a und 2b nur um ein bevorzugtes Ausführungsbeispiel handelt. So kann im einfachsten Fall auch ein separat aktuiertes Ventil zur Umschaltung zwischen den zwei Betriebszuständen vorgesehen sein, beispielsweise in Form eines Schiebers, wobei es auch möglich ist, den Schieber durch Einstellung der Drehzahl des Umluftgebläses zu verstellen oder durch die Wahl der Anzahl der in Betrieb gesetzten Umluftgebläse.

Nicht dargestellt in den Fig. 2a und 2b sind Rückstellmittel für den als Faltenbalg ausgebildeten Ansaugkanal 19. Dieser kann an sich federnd ausgebildet sein oder diesem können beispielsweise mindestens eine separate Feder zugeordnet sein, die dafür Sorge trägt, dass der Ansaugkanal 19 im ersten Betriebszustand (wieder) in Richtung Katalysator 16 zurückverstellt wird.

In den Fig. 3a und 3b ist eine alternative Ausführungsform der Anordnung in der Blackbox 5 gemäß Fig. 1 dargestellt. Hier arbeitet das Umluftgebläse 15 in Richtung Katalysator 16 blasend bzw. drückend. In Fig. 3a ist ein erster Betriebszustand dargestellt, aus dem die unmittelbar aus dem Umlufterzeugerraum 15 angesaugte Luft bzw. das Dekontaminationsmittel durch eine erste Auslassöffnung 23 zwischen einen als Faltenbalg ausgebildeten Ausblaskanal 24 an dem Katalysator 16 vorbei in Richtung zu dem dekontaminierendem Raum, in dem gezeigten Ausführungsbeispiel über einen Hochleistungsschwebstofffilter 17 strömen kann. In dem in Fig.3a gezeigten Ausführungsbeispiel wird in dem ersten Betriebszustand von dem Gebläse 15 ein geringerer Gasvolumenstrom gefördert als in dem zweiten in Fig. 3b gezeigten Betriebszustand. Dort ist zu erkennen, dass der Ausblaskanal 24 von dem Gebläse 15 weggedrückt ist in Richtung Katalysator 16 und somit die erste Ausblasöffnung 23 verschließt. Der ausgeblasene Gasvolumenstrom strömt durch eine zweite Ausblasöffnung 25, die von dem Ausblaskanal 24 begrenzt ist in den Katalysator 16 hinein und von diesem (fakultativ Ober den Hochleistungsschwebstofffilter 17) hin zu dem zu dekontaminierenden Raum, über die nicht dargestellte Membran 7.

In den Fig. 3a und 3b ist eine Hülle 26 gezeigt, die Teil der Gasleitmittel ist und die den Katalysator 16 umgibt. Die Hülle 26 trägt dafür Sorge, dass insbesondere im ersten Betriebszustand der am Katalysator 16 vorbeigeblasene Gasvolumenstrom in Richtung zu dem dekontaminierenden Raum geleitet wird.

In Fig. 3b ist zu erkennen, dass der Ausblaskanal 24 mit einer sich winklig, hier senkrecht zur Ausblasrichtung erstreckenden Anblasfläche 27 versehen ist, gegen die ein Teil des von dem Gebläse 15 geförderten Gasvolumenstroms gedrückt wird und somit den Ausblaskanal 24 in Richtung Katalysator 16 drückt.

### Bezugszeichen

- 1: Dekontaminationsanordnung
- 2: isolator
- 3: zu dekontaminierender Raum
- 4: Umlufterzeugerraum
- 5: "Blackbox"
- 6: laminare Luftströmung
- 7: Membran
- 8: Zwischenraum
- 9: Pfeilrichtungen (Strömungsrichtungen)
- 10: Strömungskanal
- 11: Frischluftleitung
- 12: Abluftleitung
- 13: Dekontaminationseinrichtung (Verdampfer)
- 14: Auslass der Dekontaminationseinrichtung
- 15: Umluftgebläse
- 16: Katalysator
- 17: Hochleistungsschwebstofffilter
- 18: Gasleitmittel
- 19: Ansaugkanal
- 20: erste Ansaugöffnung
- 21: zweite Ansaugöffnung
- 22: Ansaugfläche
- 23: erste Ausblasöffnung
- 24: Ausblaskanal
- 25: zweite Ausblasöffnung
- 26: Hülle
- 27: Anblasfläche

## Patentansprüche

1. Dekontaminationsanordnung, insbesondere für pharmatechnische Anwendungen, mit einem zu dekontaminierenden Raum (3), insbesondere einem Isolatorraum sowie mit mindestens einem oberhalb des zu dekontaminierenden Raums (3) angeordneten Umlufterzeugerraum (4) mit darin angeordnetem Gebläse zum Erzeugen eines Gasumluftvolumenstroms zwischen dem Umlufterzeugerraum (4) und dem zu dekontaminierenden Raum (3), und mit einer innerhalb des Umlufterzeugerraums (4) angeordneten und/oder einen Auslass für Dekontaminationsmittel in den Umlufterzeugerraum (4) aufweisenden, bevorzugt einen Dekontaminationsmittelverdampfer umfassenden, Dekontaminationseinrichtung (13) zur Abgabe von Dekontaminationsmittel in den Umlufterzeugerraum (4), wobei in dem Umlufterzeugerraum (4) ein Katalysator (16) zum chemischen Aufspalten des Dekontaminationsmittels vorgesehen ist, und dass das Dekontaminationsmittel mittels des Gebläses durch den Katalysator (16) förderbar ist, und dass dem Gebläse Gasleitmittel (18) zugeordnet sind, die zwischen einem ersten Betriebszustand, in dem zur Verteilung des von der Dekontaminationseinrichtung (13) abgegebenen Dekontaminationsmittels zumindest der größte Teil des mittels des Gebläse geförderten Gasumluftvolumenstroms am Katalysator (16) vorbei und einem zweiten Betriebszustand bei dem zur Aufspaltung des Dekontaminationsmittels zumindest der größte Teil des mittels des Gebläses geförderten Gasumluftvolumenstroms durch den Katalysator (16) hindurch förderbar ist, umschaltbar sind, und wobei dem mindestens einen Gebläse eine Steuereinrichtung zugeordnet ist, die das mindestens eine Gebläse (15) derart ansteuernd ausgebildet ist, dass im ersten Betriebszustand ein kleinerer Gasvolumenstrom gefördert wird als im zweiten Betriebszustand,
**dadurch gekennzeichnet,**
**dass** dem Gebläse (15) ein Gasleitmittelabschnitt zugeordnet ist, der von dem Gebläse im zweiten Betriebszustand ansaugbar ist, so dass das mindestens eine Gebläse gasleitend mit dem Katalysator (16) verbunden ist,
oder
**dass** dem Gebläse ein Gasleitmittelabschnitt zugeordnet ist, der von dem Gebläse im zweiten Betriebszustand wegdrückbar ist, so dass das das mindestens eine Gebläse gasleitend mit dem Katalysator (16) verbunden ist.

2. Dekontaminationsanordnung nach Anspruch 1,
**dadurch gekennzeichnet,**
**dass** das mindestens eine Gebläse in dem ersten Betriebszustand das Gas aus dem Umlufterzeugerraum (4) am Katalysator (16) vorbei saugend oder drückend und in dem zweiten Betriebszustand durch den Katalysator (16) hindurch saugend oder drückend angeordnet ist.

3. Dekontaminationsanordnung nach einem der Ansprüche 1 oder 2,
**dadurch gekennzeichnet,**
**dass** die Gasleitmittel (18), insbesondere vollständig, im Umlufterzeugerraum (4) angeordnet sind.

4. Dekontaminationsanordnung nach einem der vorhergehenden Ansprüche,
**dadurch gekennzeichnet,**
**dass** der in den Umlufterzeugerraum (4) ausmündende Auslass für Dekontaminationsmittel beabstandet ist von einer im Umlufterzeugerraum (4) angeordneten Ansaugöffnung des mindestens einen Gebläses.

5. Dekontaminationsanordnung nach einem der vorhergehenden Ansprüche,
**dadurch gekennzeichnet,**
**dass** eine erste Ansaugöffnung (20) der Gasleitmittel (18) durch die Gas mittels des mindestens einen Gebläses im ersten Betriebszustand ansaugbar ist und/oder eine zweite Ansaugöffnung (21), durch die Gas mittels des mindestens einen Gebläses im zweiten Betriebszustand ansaugbar ist im Umlufterzeugerraum (4) ausgebildet sind/ist.

6. Dekontaminationsanordnung nach einem der vorhergehenden Ansprüche,
**dadurch gekennzeichnet,**
**dass** eine erste Ausblasöffnung (23) der Gasleitmittel (18) durch die Gas mittels des mindestens einen Gebläses im ersten Betriebszustand ausblasbar ist und/oder eine zweite Ausblasöffnung (25), durch die Gas mittels des mindestens einen Gebläses im zweiten Betriebszustand ausblasbar ist im Umlufterzeugerraum (4) ausgebildet sind/ist.

7. Dekontaminationsanordnung nach einem der vorhergehenden Ansprüche,
**dadurch gekennzeichnet,**
**dass** im ersten Betriebszustand das mindestens eine Gebläse (15) mit geringerer Drehzahl betreibbar ist als im zweiten Betriebszustand und/oder dass im ersten Betriebszustand eine geringere Anzahl von Gebläsen (15) betreibbar sind als im zweiten Betriebszustand.

8. Dekontaminationsanordnung nach einem der vorhergehenden Ansprüche,
**dadurch gekennzeichnet,**
**dass** der zu dekontaminierende Raum (3) über mindestens einen zwischen Doppelglasscheiben ausgebildeten Umluftkanal mit dem Umlufterzeugerraum (4) verbunden ist.

9. Dekontaminationsanordnung nach einem der vorhergehenden Ansprüche,
**dadurch gekennzeichnet,**
**dass** der von dem Gebläse (15) im zweiten Betriebszustand ansaugbare Gasleitmittelabschnitt ein Ansaugkanalabschnitt der Gasleitmittel (18) ist oder dass der von dem Gebläse im zweiten Betriebszustand wegdrückbare Gasmittelendabschnitt ein Ausblaskanalabschnitt der Gasleitmittel (18) ist.

10. Dekontaminationsanordnung nach einem der vorhergehenden Ansprüche,
**dadurch gekennzeichnet,**
**dass** der Gasleitmittelabschnitt von dem Gebläse (15) im zweiten Betriebszustand entgegen der Kraft von Rückstellmitteln, bevorzugt mindestens einer Feder, ansaugbar oder wegdrückbar ist.

11. Verfahren zum Betreiben einer Dekontaminationsanordnung (1) nach einem der vorhergehenden Ansprüche, wobei in einem ersten Betriebszustand ein von dem mindestens einen Gebläse geförderter Gasvolumenstrom, zumindest größten Teils, an dem im Umlufterzeugerraum (4) angeordneten Katalysator (16) vorbei gefördert wird, und in einem zweiten Betriebszustand zur Aufspaltung des Dekontaminationsmittels zumindest der größte Teil des mittels des Gebläses geförderten Gasvolumenstroms durch den Katalysator (16) hindurchgefördert wird,
**dadurch gekennzeichnet,**
**dass** mittels des von dem mindestens einen Gebläse geförderten Gasvolumenstroms im zweiten Betriebszustand ein Gasleitmittelabschnitt zum Leiten zumindest einen Teils des Gasvolumenstroms durch den Katalysator (16) angesaugt oder weggedrückt wird.

12. Verfahren nach Anspruch 11,
**dadurch gekennzeichnet,**
**dass** von dem mindestens einen Gebläse im zweiten Betriebszustand ein größerer Gasvolumenstrom als im ersten Betriebszustand gefördert wird.

13. Verfahren nach einem der Ansprüche 11 oder 12,
**dadurch gekennzeichnet,**
**dass** das mindestens eine Gebläse, insbesondere zumindest über den größten Zeitabschnitt, während der Konditionierungsphase, in der Dekontaminationsmittel, bevorzugt in den Umlufterzeugerraum (4), zugeführt wird und/oder während einer Einwirkphase während derer das Dekontaminationsmittel seine dekontaminierende Wirkung entfaltet eingeschaltet wird oder bleibt.

14. Verfahren nach einem der Ansprüche 11 bis 13,
**dadurch gekennzeichnet,**
**dass** der Gasleitmittelabschnitt im zweiten Betriebszustand entgegen einer Rückstellkraft von Rückstellmitteln angesaugt oder weggedrückt wird.

## Claims

1. A decontamination system, more particularly for pharmatechnical applications with a room (3) to be decontaminated, more particularly an isolation room as well as, above the room to be decontaminated (3), at least one air flow generator room (4) with fans arranged therein for producing a gas circulating volume flow between the air flow generator room (4) and the room to be decontaminated (3), and a decontamination unit (13) which is arranged within the air flow generator room (4) and/or has an outlet for decontamination agents into the air flow generator room(4) and preferably comprises a decontamination agent evaporator, for releasing decontamination agents into the air flow generator room (4), wherein a catalyser (16) for chemically breaking down the decontamination agent is provided in the air flow generator room (4), and wherein the decontamination agent can be moved by the fan through the catalyser (16) and gas directing means (18) are assigned to the fan which can be switched between a first operating mode, in which in order to distribute the decontamination agent released from the decontamination unit (13) at least the greater part of the gas circulating volume flow moved by the fan can bypass the catalyser (16), and a second operating mode in which for breaking down the decontamination agent the greater part of the gas circulating volume flow moved by the fan passes through the catalyser (16), and wherein a control unit is assigned to at least one fan which is designed to control the at least one fan (15) in such a way that in the first operating mode a smaller gas volume flow is moved than in the second operating mode,
**characterised in that**
a section of the gas directing means is assigned to the fan (15) which can be drawn in by suction by the fan in the second operating mode so that at least one fan is connected to the catalyser (16) is a gas-directing manner,
or **in that**
a section of the gas directing means is assigned to the fan which can be repelled by the fan in the second operating mode so that the at least one fan is connected to the catalyser (16) in a gas-directing manner.

2. The decontamination system according to claim 1
**characterised in that**
the at least one fan is arranged in the first operating mode so that it draws or pushes the gas from the air flow generator room (4) past the catalyser (16), and in the second operating mode draws or pushes it through the catalyser (16).

3. The decontamination system according to any one of claims 1 or 2 **characterised in that**
the gas directing means (18) are arranged, more particularly fully, in the air flow generator room (4).

4. The decontamination system according to any one of the preceding claims
**characterised in that**
the outlet for decontamination agent opening into the air flow generator room (4) is at a distance from a suction opening, arranged in the air flow generator room (4), of the at least one fan.

5. The decontamination system according to any one of the preceding claims
**characterised in that**
a first suction opening (20) of the gas directing means (18) through which gas can be drawn in by means of the at least one fan in a first operating mode and/or a second suction opening (21) through which gas can be drawn in by means of the at least one fan in the second operating mode is/are provided in the air flow generator (4).

6. The decontamination system according to any one of the preceding claims
**characterised in that**
the first blowing-out opening (23) of the gas distribution means (18) through which gas can be blow out by means of the at least one fan in the first operating mode and/or a second blowing-out opening (25) through which the gas can be blown out by means of the at least one fan in the second operating mode is/are provided in the air flow generator room (4).

7. The decontamination system according to at least one of the preceding claims
**characterised in that**
in the first operating mode the at least one fan (15) can be operated at a lower speed than in the second operating mode and/or **in that** in the first operating mode a smaller number of fans (15) can be operated than in a second operating mode.

8. The decontamination system according to any one of the preceding claims
**characterised in that**
the room to be decontaminated (3) is connected to the air flow generator room (4) by means of a circulating air channel formed between double glass panes.

9. The decontamination system according to any one of the preceding claims
**characterised in that**
the section of the gas directing means subject to suction by the fan (15) in the second operation mode is a suction channel section of the gas directing means (18) or that the section of the gas directing means that can be repelled by the fan in the second operating mode is a blowing-out channel section of the gas distribution means (18).

10. The decontamination system according to any one of the preceding claims
**characterised in that**
the section of the gas directing means by the fan (15) can be drawn in or repelled in the second operating mode against the force of restoring means, preferably at least one spring.

11. A method of operating a decontamination system (1) according to any one of the preceding claims wherein in a first operating mode a gas volume flow moved by the at least one fan by-passes, at least to a greater extent, the catalyser (16) arranged in the air flow generator room (4), and in a second operating mode, in order to break down the decontamination agent at least the greater part of the gas volume flow moved by the fan passes through the catalyser (16),
**characterised in that**
by means of the gas volume flow moved by the at least one fan, in the second operating mode a section of the gas directing means is drawn in or is repelled for distributing at least part of the gas volume flow through the catalyser (16).

12. The method according to claim 11
**characterised in that**
in the second operating mode a larger gas volume flow is moved by the at least one fan than in the first operating mode.

13. The method according to any one of claims 11 or 12 **characterised in that**
that the at least one fan is or remains switched on, particularly at least over the greater period of time, during the conditioning phase, in which decontamination agent is preferably supplied to the air flow generator room (4) and/or during an exposure phase during which the decontamination agent develops its decontaminating effect.

14. The method according to any one of claims 11 to 13 **characterised in that**
in the second operating mode the section of the gas directing means is drawn in or is repelled against a restoring force of restoring means.

## Revendications

1. Dispositif de décontamination, en particulier pour des utilisations pharmacotechniques, présentant un espace (3) à décontaminer, en particulier un espace d'isolement, ainsi qu'au moins un espace (4) de génération d'une circulation de gaz disposé au-dessus de l'espace à décontaminer (3), dans lequel sont disposés des ventilateurs pour la génération d'un flux volumique de gaz entre l'espace (4) de génération d'une circulation de gaz et l'espace (3) à décontaminer, et un dispositif de décontamination (13) disposé à l'intérieur de l'espace (4) de génération d'une circulation de gaz et/ou présentant une sortie pour l'agent de décontamination dans l'espace (4) de génération d'une circulation de gaz, comprenant de préférence un évaporateur d'agent de décontamination, pour le déchargement d'agent de décontamination dans l'espace (4) de génération d'une circulation de gaz, un catalyseur (16) pour la dissociation chimique de l'agent de décontamination étant disposé dans l'espace (4) de génération d'une circulation de gaz et l'agent de décontamination pouvant être transporté au moyen du ventilateur au travers du catalyseur (16) et des moyens de guidage (18) de gaz, qui sont commutables entre un premier état de fonctionnement, dans lequel, pour la répartition de l'agent de décontamination déchargé par le dispositif de contamination (13), au moins la plus grande partie du flux volumique de gaz transporté au moyen du ventilateur est guidée en contournant le catalyseur (16), et un deuxième état de fonctionnement, dans lequel, en vue de la dissociation de l'agent de décontamination, au moins la plus grande partie du flux volumique de gaz transporté par le ventilateur peut être transportée au travers du catalyseur (16), étant associés au ventilateur et un dispositif de commande étant associé audit au moins un ventilateur, qui est conçu pour commander ledit au moins un ventilateur (15) de manière telle qu'un flux volumique de gaz transporté dans le premier état de fonctionnement est plus petit que celui transporté dans le deuxième état de fonctionnement,
**caractérisé en ce**
**qu'**une section de moyen de guidage du gaz, qui peut être aspirée par le ventilateur dans le deuxième état de fonctionnement, est associée au ventilateur (15) de telle manière qu'au moins un ventilateur est relié au catalyseur (16) de manière à guider le gaz,
ou
en ce qu'une section de moyen de guidage du gaz, qui peut être repoussée par le ventilateur dans le deuxième état de fonctionnement, est associée au ventilateur de telle manière que ledit au moins un ventilateur est relié au catalyseur (16) de manière à guider le gaz.

2. Dispositif de décontamination selon la revendication 1,
**caractérisé en ce**
**que** ledit au moins un ventilateur est disposé, dans le premier état de fonctionnement, de manière à aspirer ou à souffler le gaz provenant de l'espace (4) de génération d'une circulation de gaz contournant le catalyseur (16), et, dans le deuxième état de fonctionnement, de manière à aspirer ou à souffler le gaz au travers du catalyseur (16).

3. Dispositif de décontamination selon l'une quelconque des revendications 1 ou 2,
**caractérisé en ce**
**que** les moyens de guidage (18) du gaz sont disposés, en particulier complètement, dans l'espace (4) de génération d'une circulation de gaz.

4. Dispositif de décontamination selon l'une quelconque des revendications précédentes,
**caractérisé en ce**
**que** la sortie pour l'agent de décontamination débouchant dans l'espace (4) de génération d'une circulation de gaz est écartée d'un orifice d'aspiration dudit au moins un ventilateur disposé dans l'espace (4) de génération d'une circulation de gaz.

5. Dispositif de décontamination selon l'une quelconque des revendications précédentes,
**caractérisé en ce**
**qu'**un premier orifice d'aspiration (20) des moyens de guidage (18) du gaz, au travers duquel du gaz peut être aspiré au moyen dudit au moins un ventilateur dans le premier état de fonctionnement, et/ou un deuxième orifice d'aspiration (21), au travers duquel du gaz peut être aspiré au moyen dudit au moins un ventilateur dans le deuxième état de fonctionnement, est/sont réalisé(s) dans l'espace (4) de génération d'une circulation de gaz.

6. Dispositif de décontamination selon l'une quelconque des revendications précédentes,
**caractérisé en ce**
**qu'**un premier orifice de refoulement (23) des moyens de guidage (18) du gaz, au travers duquel du gaz peut être refoulé au moyen dudit au moins un ventilateur dans le premier état de fonctionnement, et/ou un deuxième orifice de refoulement (25), au travers duquel du gaz peut être refoulé au moyen dudit au moins un ventilateur dans le deuxième état de fonctionnement, est/sont réalisé(s) dans l'espace (4) de génération d'une circulation de gaz.

7. Dispositif de décontamination selon l'une quelconque des revendications précédentes,
**caractérisé en ce**
**que**, dans le premier état de fonctionnement, ledit au moins ventilateur (15) peut être entraîné à une vitesse de rotation inférieure à celle dans le deuxième état de fonctionnement et/ou en ce que, dans le premier état de fonctionnement, le nombre de ventilateurs (15) pouvant être entraînés est inférieur à celui dans le deuxième état de fonctionnement.

8. Dispositif de décontamination selon l'une quelconque des revendications précédentes,
**caractérisé en ce**
**que** l'espace (3) à décontaminer est relié à l'espace (4) de génération d'une circulation de gaz via au moins un canal de circulation formé entre des doubles vitrages.

9. Dispositif de décontamination selon l'une quelconque des revendications précédentes,
**caractérisé en ce**
**que** la section de moyen de guidage du gaz pouvant être aspirée par le ventilateur (15) dans le deuxième état de fonctionnement est une section de canal d'aspiration des moyens de guidage (18) du gaz ou en ce que la section de moyen de guidage du gaz pouvant être repoussée par le ventilateur dans le deuxième état de fonctionnement est une section de canal de soufflage du moyen de guidage (18) du gaz.

10. Dispositif de décontamination selon l'une quelconque des revendications précédentes,
**caractérisé en ce**
**que** la section de moyen de guidage du gaz peut être aspirée ou repoussée par le ventilateur (15) dans le deuxième état de fonctionnement contre une force de moyens de rappel, de préférence au moins un ressort.

11. Procédé pour le fonctionnement d'un dispositif de décontamination (1) selon l'une quelconque des revendications précédentes, un flux volumique de gaz, au moins sa plus grande partie, transporté par ledit au moins un ventilateur, dans un premier état de fonctionnement, étant guidé en contournant le catalyseur (16) disposé dans l'espace (4) de génération d'une circulation de gaz et, dans un deuxième état de fonctionnement, pour la dissociation de l'agent de décontamination, au moins la plus grande partie du flux volumique de gaz transporté au moyen du ventilateur étant transportée au travers du catalyseur (16),
**caractérisé en ce**
**qu'**une section de moyen de guidage du gaz, en vue du guidage d'au moins une partie du flux volumique de gaz au travers du catalyseur (16), peut être aspirée ou repoussée au moyen du flux volumique de gaz transporté par ledit au moins un ventilateur dans le deuxième état de fonctionnement.

12. Procédé selon la revendication 11,
**caractérisé en ce**
**que**, dans le deuxième état de fonctionnement, un flux volumique de gaz transporté par ledit au moins un ventilateur est plus grand que celui transporté dans le premier état de fonctionnement.

13. Procédé selon l'une quelconque des revendications 11 ou 12,
**caractérisé en ce**
**que** ledit au moins un ventilateur est ou reste commuté, en particulier au moins pendant la période la plus longue, pendant la phase de conditionnement, lors que laquelle de l'agent de décontamination est alimenté, de préférence dans l'espace (4) de génération d'une circulation de gaz, et/ou pendant une phase d'action, pendant laquelle l'agent de décontamination déploie son action de décontamination.

14. Procédé selon l'une quelconque des revendications 11 à 13,
**caractérisé en ce**
**que** la section du moyen de guidage du gaz est aspirée ou repoussée, dans le deuxième état de fonctionnement, contre une force de rappel de moyens de rappel.
